(19) Europäisches Patentamt
European Patent Office
Office européen des brevets

(11) EP 0 714 914 B1

# (12) EUROPÄISCHE PATENTSCHRIFT

(45) Veröffentlichungstag und Bekanntmachung des Hinweises auf die Patenterteilung:
**16.06.1999 Patentblatt 1999/24**

(51) Int Cl.6: **C08B 31/04**, A61L 15/00

(21) Anmeldenummer: **95118224.5**

(22) Anmeldetag: **20.11.1995**

(54) **Verfahren zur Herstellung quellbarer Stärkeester**

Process for preparing swellable starch ester

Procédé de préparation d'ester d'amidon gonflable

(84) Benannte Vertragsstaaten:
**AT BE CH DE DK ES FR GB GR IE IT LI NL PT SE**

(30) Priorität: **30.11.1994 DE 4442606**

(43) Veröffentlichungstag der Anmeldung:
**05.06.1996 Patentblatt 1996/23**

(73) Patentinhaber: **Degussa Aktiengesellschaft
60311 Frankfurt (DE)**

(72) Erfinder:
- **Buchholz, Stefan Dr.
D-63456 Hanau (DE)**
- **Dorn, Klaus Dr.
D-63457 Hanau (DE)**
- **Eurich, Thomas
D-60388 Frankfurt (DE)**

(56) Entgegenhaltungen:
**CH-A- 631 085**
**DE-A- 2 533 005**
**US-A- 2 461 139**

- **DATABASE WPI Week 8201 Derwent Publications Ltd., London, GB; AN 488E & JP-A-56 152 806 (KURARAY KK) , 26.November 1981**
- **DATABASE WPI Week 8201 Derwent Publications Ltd., London, GB; AN 826E & JP-A-56 155 203 (KURARAY KK) , 1.Dezember 1981**

Bemerkungen:
Die Akte enthält technische Angaben, die nach dem Eingang der Anmeldung eingereicht wurden und die nicht in dieser Patentschrift enthalten sind.

EP 0 714 914 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung eines Absorptionsmaterial, das überwiegend auf nachwachsenden Rohstoffen basiert und gegenüber den derzeit als Absorbermaterial überwiegend verwendeten Polyacrylaten eine deutlich verbesserte biologische Abbaubarkeit aufweist.

Insbesondere betrifft die Erfindung ein Verfahren zur Herstellung eines quellbaren Stärkeesters, der eine vergleichsweise hohe Aufnahmekapazität aufweist und der im Vergleich zu anderen Absorbermaterialien auf Polysaccharidbasis nur eine sehr geringe Neigung zum Gelblocking zeigt.

Die weitaus meisten der heute verwendeten Absorptionsmaterialien, häufig auch als Superabsorber bezeichnet, bestehen aus schwach vernetzten Polyacrylaten und sind somit nur zu einem sehr geringen Teil oder überhaupt nicht abbaubar (s. z. B. Stegman et al., Waste Manage. Res. 11 (1993) 155).

Neben den reinen Polyacrylaten gibt es auch auf Stärke gepfropfte Polyacrylate (DE-A 26 12 846). Der Stärkegehalt dieser Produkte ist mit bis zu 25 % jedoch gering. Bei höheren Stärkegehalten wird eine deutliche Verschlechterung der Absorptionseigenschaften beobachtet. Aufgrund des Polyacrylatgehaltes ist die biologische Abbaubarkeit auch dieser Produkte gering.

Ebenso können bis zu ca. 25 % eines zumindest begrenzt wasserlöslichen Polysaccharides in einen vernetzten Polyacrylatsuperabosorber eingearbeitet werden, indem das Polysaccharid während der Polymerisation des Acrylates in das Reaktionsgemisch eingebracht wird (DE-A 40 29 591, DE-A 40 29 592, DE-A 40 29 593).

US-A 5,079,354 beschreibt ein Absorbermaterial auf Basis von Carboxymethylstärke, also eines Stärkeethers, das durch Umsetzung von Stärke mit Chloressigsäure hergestellt wir. Bei diesem Prozeß wird eine äquivalente Menge Natriumchlorid bezogen auf die eingesetzte Chloressigsäure freigesetzt, was aus ökologischen Gründen unerwünscht ist. Ferner ist bekannt, daß veretherte Polysaccharide bei hohen Substitutionsgraden nur schlecht biologisch abbaubar sind (Mehltretter et al. J. Am. Oil Chem. Soc. 47 (1970) 522).

Aus der DE-A 31 32 976 ist die Verwendung von Stärkesuccinatderivaten als Streckmittel für trockene, feste in Wasser quellbare Absorptionsmittel auf Basis eines ionisch komplexierten anionischen Polyelektrolyten, z. B. Polyacrylsäure/Aluminiumkation-Komplex, bekannt. Dabei werden in der DE-A 31 32 976 eine ganze Reihe von in Frage kommenden Streckmitteln aufgezählt, die auch die Absorptionseigenschaften der sie enthaltenden Mischungen mit z. B. Polyacrylsäure verbessern. So gehören zu den Streckmitteln, welche bevorzugt mit stärker oberflächenbehandelten ionisch komplexierten Polyelektrolyten zu vermischen sind, vor allem Natriumcarboxymethylzellulose, Methylzellulose, fein zerteilter Attapulgitton, Mischungen von Natriumcarboxymethylzellulose mit Attapulgitton oder mit grobem oder feinem Montmorillonitton, kaltwasserdispergierbare Wachsmaisstärke und Stärkesuccinatderivate. In Beispielen zeigen Mischungen aus 40 % walzengetrocknetem Stärkesuccinatderivat und 60 % Polyelektrolyt nur eine geringfügige Verbesserung der Blut-Salz-Druck-Retention des reinen Polyelektrolyten ohne Streckmittel. Für ein reines walzengetrocknetes Stärkesuccinatderivat wird zu Vergleichszwecken ein Wert von 4,0 g/g als Ergebnis des Blut-Salz-Druck-Retention-Tests angegeben. Dieser Wert liegt bei etwa 1/5 des reinen Polyelektrolyten.

Die EP-A 0 603 837 beschreibt die Herstellung von Stärkeestern unter Verwendung von organischen Säureanhydriden. Hierzu läßt man Stärke diverser Herkunft in einem einstufigen, wässrigen Verfahren mit organischen Säureanhydriden der allgemeinen Formel I reagieren,

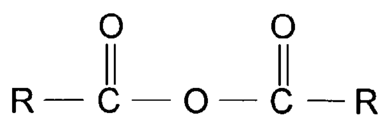

I

worin R alkyl, aryl, alkenyl, alkaryl oder aralkyl mit 1 bis 7 C-Atomen bedeutet, unter bestimmten pH-, Temperatur- und Konzentrationsbedingungen. Zu beispielhaft in der Beschreibung der EP-A 0 603 837 aufgezählten Stärkeestern gehören Stärkeacetat, -propionat, -butyrat, -hexanoat, -benzoat oder auch gemischte Stärkeacetate/propionate. In den Beispielen der EP-A 0 603 837 wird die Verwendung von Propionsäureanhydrid, Acetanhydrid und/oder Buttersäureanhydrid offenbart. Mit dem in der EP-A 0 603 837 beschriebenen Verfahren gelingt es, die Probleme zu umgehen, die sich sonst beim Einsatz größerer Mengen Anhydrid ergeben haben, wie etwa das Quellen oder Gelantinieren der Stärke und Probleme beim Abtrennen der Stärkeester aus der Reaktionsmischung. Das Produkt wird nämlich durch die Umsetzung hydrophob und daher in einfacher Weise abfiltrierbar.

Aus der WO 93/01217 (PCT/EP 92/01553) ist ein Verfahren zur Herstellung von Stärkeestern für klinische, insbesondere parenterale Anwendung bekannt. Zur Herstellung von wasserlöslichen, physiologisch verträglichen Stärkeestern wird Stärke durch Säurehydrolyse oder Enzymhydrolyse in ein Teilhydrolysat mit einem mittleren $M_w$ im Bereich von 10.000 bis 500.000 Dalton überführt, dieses Teilhydrolysat in wässriger Lösung mit dem Anhydrid oder Halogenid einer aliphatischen Monocarbonsäure mit 2 - 4 Kohlenstoffatomen oder einer aliphatischen Dicarbonsäure mit 3 - 6 Kohlenstoffatomen oder Gemischen davon und einem Alkalisierungsmittel bis zu einer molaren Substitution im Bereich von 0,1 bis 1,0 Mal/Mol acyliert.

Die Stärkeester gemäß der WO 93/01217 sind sehr gut wasserlöslich, was für die parenterale Anwendung zwingend erforderlich ist.

Als Blutplasmaverdicker ist beispielsweise dieser Anmeldung gemäß Beispielen eine durch Umsetzung eines Wachsmaisstärke-Teilhydrolysats mit Essigsäureanhydrid erhältliche Acetylstärke entnehmbar.

Obwohl weitere Stärken und Anhydride in der WO 93/01217 genannt werden, u. a. Anhydride oder Halogenide einer Dicarbonsäure, z. B. Bernsteinsäure oder Maleinsäure, lehrt die in Rede stehende Druckschrift ausschließlich die Herstellung wasserlöslicher, physiologisch verträglicher Stärkeester.

Es hat auch bereits Versuche gegeben, biologisch abbaubare Superabsorber zu schaffen. So kennt man aus der DE-A 42 06 857 ein Absorptionsmittel, das aus einer auf speziellen nachwachsenden Polysaccharid-Rohstoffen basierenden Komponente, einem speziellen wasserquellbaren Polymer, einem Matrixmaterial, einem ionischen oder kovalenten Vernetzer und einem Reaktivzusatz besteht. Die auf nachwachsenden Polysaccharid-Rohstoffen basierende Komponente umfaßt z. B. Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke. Ferner ist es aus der DE-A 42 06 857 bekannt, daß die aufgeführten Polymeren durch eine Vernetzung modifiziert werden können, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.

Die Umsetzung der Polymeren kann mit ionischen Vernetzern wie z. B. Calcium-, Aluminium-, Zirkon und Eisen(III)-Verbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Glykoldiglycidylether, Glykoldi- oder -triglycidylether und Epichlorhydrin, mit Säureanhydriden wie Bernsteinsäureanhydrid und Maleinsäureanhydrid, mit Aldehyden und mehrfunktionellen (aktivierten) Olefinen wie Bis-(acrylamido)-essigsäure und Methylenbisacrylamid möglich. Ebenso kommen natürlich Derivate der genannten Verbindungsklassen in Betracht sowie heterofunktionelle Verbindungen mit verschieden funktionellen Gruppen der oben genannten Verbindungsklassen.

Obwohl die vorgestellten und anhand von Beispielen belegten Systeme auf Basis von Natriumcarboxymethylcellulose in Verbindung mit Natriumpolyacrylat in verschiedenen Tests recht günstige Absorptionseigenschaften aufweisen, ist der Druckschrift in Bezug auf die biologische Abbaubarkeit, mit Ausnahme des Umstandes, daß letztere einfach behauptet wird, nichts Näheres entnehmbar.

Es ist jedoch bekannt, daß Polyacrylate praktisch gar nicht (R. Stegmann et al. Waste Water Res. 11(2) (1993) S. 155) und Carboxymethylcellulose, ein Polysaccaridether nur sehr schlecht biologisch abbaubar ist (4,6 % nach 5 Tagen; M. Seekamp, Textilveredlung 25 (1990) S. 125).

Aus der JP 56 152 806 ist ein Verfahren zur Herstellung von Superabsorbern bekannt, bei welchem Stärke in einer wässrigen Reaktion mit Carbonsäureanhydriden umgesetzt wird. Dabei wird in einem ersten Schritt aus der Stärke ein Alkoholat erzeugt, welches isoliert und im wasserfreien Zustand mit dem Anhydrid verestert wird. Hierzu werden organische Lösungsmittel als Quellmittel eingesetzt, beispielsweise Benzolgemische, Toluol oder Xylol.

Angesichts des vorstehend diskutierten Standes der Technik ist es Aufgabe der Erfindung gewesen, ein einfaches Verfahren zur Herstellung eines Absorptionsmaterials mit einer den derzeit überwiegend verwendeten Polyarcylaten gegenüber wesentlich verbesserten biologischen Abbaubarkeit anzugeben.

Gelöst wird diese Aufgabe durch ein Verfahren zur Herstellung eines quellbaren Stärkeesters, der zu mehr als 50 Gew.-% aus in Wasser unlöslichen Anteilen besteht und ein Rückhaltevermögen für 0,9 gew.-%ige wässrige NaCl-Lösung von > 500 % aufweist, jeweils bezogen auf das Gewicht des trockenen Stärkeesters, wobei das Rückhaltevermögen dadurch bestimmt wird, daß man 0,1 g des in einen Nylonbeutel mit 52 µm Maschenweite eingeschweißten Stärkeesters für 30 min in einer 0,9 %igen NaCl-Lösung quellen läßt, den Beutel anschließend 5 min bei 1400 rpm zentrifugiert und danach eine ggf. resultierende Gewichtszunahme gravimetrisch bestimmt, bei welchem Verfahren Stärke oder modifizierte Stärke in einer einstufigen wässrigen Reaktion mit einem Carbonsäureanhydrid oder einer Mischung von Carbonsäureanhydriden bei einem pH-Wert von 7 bis 11 und einer Temperatur von 0 bis 40 °C umgesetzt wird, wobei der pH-Wert durch Zugabe von wässriger Alkali-Lösung mit einer Konzentration von ca. 10 bis 50 Gew.-% im angegebenen Bereich gehalten wird, und wobei als Carbonsäureanhydrid ein cyclisches ungesättigtes Anhydrid oder eine Mischung von Carbonsäureanhydriden eingesetzt wird, die ein solches cyclisches ungesättigtes Anhydrid aufweist.

Im Rahmen der Erfindung wurde überraschend gefunden, daß bei der Modifizierung kaltwasserlöslicher, d. h. vorgelatinisierter Stärke ein stark quellbares aber zu mehr als 50 % unlösliches Produkte erhältlich ist. Dieses Material ist für den Einsatz als Absorptionsmaterial zu Absorption von Wasser, wäßrigen Lösungen, Dispersionen und Körperflüssigkeiten in Hygiene- und Tierhygiene, insbesondere in Windeln und Inkontinenzprodukten, sowie in Verpackungsmaterialien für Fleisch und Fisch, sowie zur Bodenverbesserung, zum Einsatz in Kulturgefäßen und als Kabelummantelungen geeignet, wobei es gleichzeitig hervorragend biologisch abbaubar ist.

Im Gegensatz zu den in der DE-A 42 06 857 als Komponente A angegebenen Substanzen, deren biologische Abbaubarkeit einfach behauptet wird, weil sie - wie es heißt - auf speziellen nachwachsenden Polysaccharid-Rohstoffen basieren, sind die Superabsorbermaterialien gemäß der vorliegenden Erfindung biologisch abbaubar, wie später eingehender an Modellverbindungen erörtert werden wird.

In bevorzugter erfindungsgemäßer Ausführungsform werden Stärkeester hergestellt, die sich durch ein Rückhaltevermögen für 0,9 gew.-%ige wässrige NaCl-Lösung von > 1500 % bezogen auf das Gewicht des trockenen, ungequollenen Stärkeesters, kennzeichnen.

Bei den erfindungsgemäß herstellbaren Stärkeestern handelt es sich um Produkte mit einem Substitutionsgrad zwischen 0,2 und 2,0, wobei der Substitutionsgrad die Anzahl der Substituenten pro Glucosering angibt. Bei den Estern kann es sich um Produkte mit nur einer Art von Estergruppe handeln oder um gemischte Ester.

Die Ester werden durch Umsetzung von Stärke mit Säureanhydriden erhalten, wobei es sich bei den Anhydriden um cyclische und/oder offenkettige Anhydride handeln kann. Erfindungsgemäß ist Maleinsäureanhydrid bevorzugt.

Aus der Umsetzung von Stärke mit offenkettigen Anhydriden resultieren prinzipiell nichtionische Stärkeester, während aus der Umsetzung von Stärke mit cylischen Anhydriden grundsätzlich ionische, carboxylatgruppentragende Stärkeester resultieren. Um die erfindungsgemäßen quellbaren, aber zu mehr als 50 % unlöslichen Absorptionsmaterialien zu erhalten, ist es ganz besonders bevorzugt, wenn das Produkt freie Carboxyl- bzw. Carboxylatgruppen enthält. Daher ist es von Vorteil, wenn es sich zumindest bei einem Teil des eingesetzten Anhydrides um ein cyclisches Anhydrid handelt.

Ferner ist ein Produkt bevorzugt, das zumindest in einem Teil der Seitengruppen Doppelbindungen trägt. Der Maleinsäureester der Stärke verkörpert diese beiden erwünschten Eigenschaften in ganz besonders bevorzugter Weise.

Bei der Umsetzung kaltwasserlöslicher, d. h. vorgelatinisierter Stärke mit Bernsteinsäureanhydrid wird z. B. ein wasserlösliches Produkt erhalten. Wahrscheinliche Ursache für die Unlöslichkeit des Maleinsäureproduktes ist daher eine Vernetzung die über eine Michael-Addition an die Doppelbindung der Maleinsäureseitengruppe erfolgt.

DE-A 42 06 857 beschreibt, wie bereits weiter oben ausgeführt, ein Absorptionsmittel, das neben dem biologisch nicht abbaubaren Polyacrylat eine Stärke bzw. ein Stärkederivat enthalten kann. In diesem Zusammenhang wird in DE-A 42 06 857 die Vernetzung von Stärke oder Stärkederivaten wie Carboxymethylstärke (also einem Stärkeether) mit Maleinsäureanhydrid zur Verringerung der Löslichkeit und einer damit verbundenen Verbesserung der Quelleigenschaften beschrieben. Demgegenüber liegt der Erfindung die Erkenntnis zugrunde, daß ohne die Verwendung von biologisch nicht abbaubarem Polyacrylat durch die Veresterung von Stärke oder Stärkederivaten mit Carbonsäureanhydriden und insbesondere mit Maleinsäureanhydrid gemäß der Gleichung

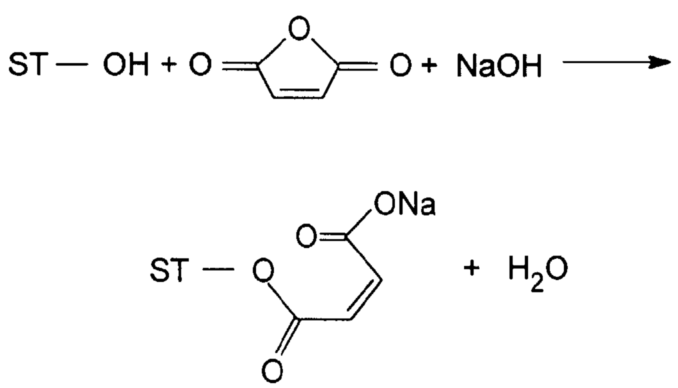

wobei ST-OH für Stärke oder ein Stärkederivat steht, ionische Gruppen in das Polysaccharidgerüst eingeführt werden können, die aufgrund des durch sie erzeugten osmotischen Druckes zu besonders günstigen Absorptions- und Quelleigenschaften führen, und daß dabei eine hervorragende biologische Abbaubarkeit erhalten bleibt.

Zur Erzielung eines vorteilhaften Quellvermögens ist neben der Einführung ionischer Gruppen auch eine Vernetzung notwendig, um das Material in eine zwar quellbare aber wasserunlösliche Form zu überführen.

Beides sowohl Veresterung zur Einführung ionischer Gruppen und Vernetzung geschieht dabei im Rahmen der Erfindung besonders vorteilhaft mit Maleinsäureanhydrid oder einer Mischung von wenigstens zwei Anhydriden, von denen eines Maleinsäureanhydrid ist, bevorzugt in einem einzigen Schritt. Wenn es aber wie gemäß der DE-A 42

06 857 an einer ausreichenden Zahl an ionischen Gruppen im Molekül fehlt, dann ergibt sich ein völlig andersartiges Produkt, das nicht die Quellbarkeit in Verbindung mit dem Retentionsvermögen der quellbaren Stärkeester der Erfindung aufweist. Zu den Carbonsäureanhydriden, die besonders vorteilhaft zusammen mit Maleinsäureanhydrid eingesetzt werden, gehören u. a. Acetanhydrid, Propionsäureanhydrid und/oder Bernsteinsäureanhydrid.

Als Stärkebasis der erfindungsgemäß herstellbaren Materialien kann im Prinzip jede native, modifizierte oder subsituierte Stärke eingesetzt werden. Derartige Stärken können aus einer beliebigen pflanzlichen Quelle isoliert worden sein, und umfassen z. B. Kartoffelstärke, Maisstärke, Weizenstärke, Wachsmaisstärke und Stärken mit hohem Amylosegehalt. Stärkemehl kann ebenfalls verwendet werden. Auch verwendet werden können modifizerte Produkte auf Basis einer der oben aufgeführten Stärken wie z. B. säurehydrolysierte Stärke, enzymhydrolysierte Stärke, Dextrine und oxidierte Stärke. Des weiteren können derivatisierte Stärken wie kationische Stärke, anionische Stärke, amphotere Stärke oder nichtionisch modifizierte Stärke wie z. B. Hydroxyethylstärke verwendet werden. Bei den verwendeten Stärken kann es sich um granuläre oder vorgelatinisierte Stärke handeln, wobei die Zerstörung der granulären Struktur thermisch, mechanisch oder chemisch erfolgen kann.

Besonders vorteilhaft für die Erfindung ist der Einsatz kaltwasserlöslicher Stärke. Hierunter versteht man insbesondere vorgelatinierte oder partiell abgebaute Stärke. Hierzu gehört u. a. Aeromyl 115 der Firma Südstärke.

Beim Verfahren gemäß der Erfindung zur Herstellung eines Stärkeesters wird - wie bereits erwähnt - Stärke oder modifizierte Stärke in einer einstufigen wässrigen Reaktion mit einem Carbonsäureanhydrid oder einer Mischung von Carbonsäureanhydriden bei einem pH-Wert von 7 bis 11 und einer Temperatur von 0 bis 40 °C umgesetzt, wobei der pH-Wert durch Zugabe von wässriger Alkali-Lösung mit einer Konzentration von ca. 10 bis 50 Gew.-% im gewünschten Bereich gehalten wird. Das Herstellverfahren ist ferner dadurch gekennzeichnet, daß als Carbonsäureanhydrid ein cyclisches ungesättigtes Anhydrid oder eine Mischung von Carbonsäureanhydriden eingesetzt wird, die ein solches cyclisches ungesättigtes Anhydrid aufweist.

Während der Reaktion des Anhydrides mit der Stärke wird der pH vorzugsweise konstant gehalten. Der pH sollte während der Reaktion zwischen 7 und 11 liegen. Bevorzugt wird ein pH Wert zwischen 8 und 9. Der pH kann im Prinzip durch Zugabe eines beliebigen alkalischen Materials konstant gehalten werden. Besonders nützlich sind Alkali- und Erdalkalihydoxide sowie die Oxide und Carbonate dieser Metalle. Beispielhaft genannt seien Natriumhydroxid, Kaliumhydroxid, Calciumhydroxid, Ammoniumhydroxid, Magnesiumhydroxid und Natriumcarbonat. Bei der Herstellung von Produkten mit einem höheren Substitutionsgrad wird bevorzugt eine Alkalilösung mit einer höheren Konzentration, ca. 10 bis 50 %ig, verwendet um eine unnötige Verdünnung des Reaktionsmediums zu vermeiden.

Gegenstände der Erfindung finden Verwendung in einer Menge von 100 Gew.-Teilen zusammen mit 0,7 - 70 Gew.-Teilen eines Antiblockingmittels auf Basis natürlicher oder synthetischer, bevorzugt hydrophiler Fasern oder Materialien mit großer Oberfläche als Superabsorber. Bevorzugt ist die Verwendung des Stärkeesters als Superabsorber zusammen mit 1 bis 5 Gew.-Teilen Kieselsäure oder Cellulosefasern als Antiblockingmittel.

Weitere Anwendung findet der Stärkeester der Erfindung als Absorptionsmaterial zur Absorption von Wasser, wäßrigen Lösungen, Dispersionen und Körperflüssigkeiten in Hygiene- und Tierhygiene, insbesondere in Windeln, Tampons und Inkontinenzprodukten sowie in Verpackungsmaterialien für Fleisch und Fisch, als Absorptionsmaterial zur Absorption von Wasser und wäßrigen Lösungen in Kulturgefäßen und zur Bodenverbesserung oder als Absorptionsmaterial zur Absorption von Wasser und wäßrigen Lösungen in Kabelummantelungen.

Die erfindungsgemäßen Materialien sind zumindest teilweise biologisch abbaubar. Für quellbare, aber wasserunlösliche Produkte gibt es z. Z. noch keinen allgemein anerkannten Test zur Bestimmung der biologischen Abbaubarkeit. Um dennoch eine zumindest qualitative Aussage treffen zu können, wurde als wasserlösliche, oligomere Modellverbindung ein Dextrinmaleat mit einem DS(th.)=1 und DS(NaOH)= 0,92 bzgl. der biologischen Abbaubarkeit untersucht. Die Modellverbindung wird im Zahn-Wellens-Test innerhalb von 28 Tagen zu 65 % und innerhalb von 42 Tagen zu 95 % abgebaut. Selbst ein Dextrinmaleat mit einem sehr hohen Substitutionsgrad, DS(th.) = 2,0 und DS (NaOH) = 1,47 wird im Zahn-Wellens-Test innerhalb von 28 Tagen zu 66 % und innerhalb von 42 Tagen zu 88 % abgebaut.

Ein vernetztes Produkt analog zu Beispiel 1, ein Stärkemaleat mit einem DS(th.) = 1,0 und DS(NaOH) = 0,81, wurde innerhalb von 28 Tagen zu 30 % abgebaut, wobei der biologische Abbau auch nach 28 Tagen noch weiter ansteigt. Der geringe Abbau des vernetzten Produktes ist wahrscheinlich auf die schlechtere Zugänglichkeit der Polymeren in dem vernetzten Produkt zurückzuführen, da in diesem die Mikroorganismen und Enzyme die Gelpartikel nur von der Oberfläche her angreifen können. Da jedoch auch nach 28 Tagen noch eine annähernd lineare Zunahme des biologischen Abbaus mit der Zeit beobachtet wird, liegt die Vermutung nahe, daß die erfindungsgemäßen Produkte und insbesondere Stärkemaleat im Prinzip vollständig biologisch abbaubar ist.

Die Vermutung wird ferner dadurch unterstützt, daß Stärkesuccinat, ein anderer anionischer Stärkeester, der jedoch wasserlöslich ist, innerhalb von 28 Tagen zu 90 % biologisch abgebaut wird.

Zum Vergleich: Für Carboxymethylstärke, einem Stärkeether mit einem theoretischen DS von 1,0 wird hingegen nach 21 Tagen nur ein biologischer Abbau von 13 % gefunden, was die literaturbekannte schlechte biologische Abbaubarkeit von Stärkeethern belegt (Mehltretter et al. J. Am. Oil Chem. Soc. 47 (1970) 522).

**Bestimmung der Substitutionsausbeute über der NaOH-Verbrauch während der Synthese**

Bei der Umsetzung von Stärke mit Maleinsäureanhydrid (MSA)in wäßriger Lösung wird pro mol Maleinsäureanhydrid ein mol NaOH verbraucht. Findet keine Addition des Maleinsäureanhydrides an die Stärke sondern eine Hydrolyse zum Dinatriummaleat statt, so werden, bei pH-Konstanthaltung, pro Mol MSA 2 Mol NaOH verbraucht. Wenn am Ende der Reaktion kein nicht reagiertes Anhydrid mehr vorliegt, läßt sich der Substitutionsgrad nach

$$DS = 1 - \frac{(\text{Verbrauch an NaOH in Mol - eingesetzte Menge MSA in Mol})}{(\text{eingesetzte Menge MSA in Mol})}$$

berechnen. Der so ermittelte Substitutionsgrad wird im folgenden mit DS(NaOH) bezeichnet.

**Methode zur Bestimmung des Rückhaltevermögens (Retentionsvermögens)**

Zur Bestimmung des Retentionsvermögens wurde ein Teebeutel-Test durchgeführt. Hierzu werden 0,10 g der Prüfsubstanz in einen Beutel aus Nylongewebe mit einer Maschenweite von 52 µm eingewogen. Der verschweißte Nylonbeutel wird in eine 0,9 %ige NaCl-Lösung gegeben und das Prüfmaterial wird 30 Minuten quellen gelassen. Anschließend wird der Beutel herausgenommen und 5 Minuten bei 1400 Upm in einem Zentrifugeneinsatz mit Siebboden geschleudert. Die Flüssigkeitsaufnahme wird gravimetrisch bestimmt und auf 1 g der zu prüfenden Substanz umgerechnet. Der so erhaltene Wert wird als Rückhaltevermögen (Retentionsvermögen) bezeichnet (abgekürzt SRV für saline retention value).

**Methode zur Bestimmung des Absorptionsvermögens mit und ohne Last**

0,5 g der Prüfsubstanz werden auf eine G3-Glasfritte mit 3 cm Durchmesser gegeben. Die Fritte ist mit einer Bürette über einen Schlauch verbunden, wobei die Bürette mit 0,9 %iger NaCl-Lösung gefüllt ist. Es wird die Flüssigkeitsmenge bestimmt, die die Prüfsubstanz in 10 Minuten aufsaugt. Während der Versuchsdauer wird die Bürette so nachgeführt, daß der Meniskus immer auf Höhe der Glasfritte ist. Der so bestimmte Wert wird auf 1 g Testsubstanz umgerechnet und mit A(NaCl) bezeichnet.

In einer anderen Ausführung wird auf die Testsubstanz ein Gewicht gestellt, das einen Druck von 20 $g/cm^2$ ausübt. Der so erhaltene Wert wird ebenfalls auf 1 g Testsubstanz umgerechnet und mit AUL(NaCL) abgekürzt (Absorption unter Last).

**Bestimmung der löslichen Anteile**

Zur Bestimmung der löslichen Anteile wird 1 g des zu charakterisierenden Produktes in 100 ml entmineralisiertes Wasser gegeben und es wird 24 Stunden bei Raumtemperatur gerührt. Die entstandenen Gelpartikel werden abzentrifugiert und es wird eine Probe von ca. 10 ml von der überstehenden Lösung abgenommen, gewogen, einrotiert, der Rückstand wird gewogen und die gemessene Menge wird auf die Gesamteinwaage an Absorbermaterial zurückgerechnet.

**Biologischer Abbau nach Zahn-Wellens**

Der Abbautest nach Zahn und Wellens ist ein Hilfsmittel, die biologische Abbaubarkeit einer Substanz bzw. eines Abwassers zu beurteilen. Er ist in quantitativer Weise nur auf wasserlösliche Substanzen anwendbar. Für nicht vollständig lösbare Substanzen gibt er nur einen qualitativen Hinweis, ob diese Substanzen prinzipiell einem biologischen Abbau zugänglich sind oder nicht.

In einem hohen 3 l-Becherglas werden ca. 2 l Biomassesuspension gerührt und über Glasfritten belüftet. Es werden 385 mg $NH_4Cl$ und 89 mg $NaH_2PO_4H_2O$ eingewogen, mit der berechneten Polymermenge versetzt und mit kaltem Leitungswasser auf ca. 2 l aufgefüllt. Der Belebtschlamm aus einer kommunalen Kläranlage wird 30 - 60 in absetzen lassen, so daß er ca. um die Hälfte eindickt. Das überstehende Wasser wird abdekantiert und der Schlamm unter Rühren und Belüften aufbewahrt. Jeweils ein Teil des Schlammes wird davon abgenommen und bei 200 Upm 5 min zentrifugiert. 24 g des zentrifugierten Schlammes auf einen 2 l-Ansatz ergeben einen Trockensubstanzgehalt von 1 g/l (± 200 %).Vor der Zugabe des Schlammes zur Polymerlösung wird der pH auf 6,5 - 7,0 eingestellt und eine Probe auf den CSB-Gehalt hin analysiert. Nachdem sich der Belebtschlamm fein verteilt hat, kann erneut eine Probe entnommen werden. Die Höhe der Flüssigkeit im Becherglas wird dann markiert. Zum Vergleich wird ein Test angesetzt, der nur Nährsalze und Belebtschlamm, jedoch kein Polymer enthält. Dieser Ansatz dient zur Ermittlung des durch den Belebtschlamm verursachten CSB. Mit einem Gummiwischer wird der am Rand des Becherglases abgesetzte

Bioschlamm täglich zurück in die Lösung gewischt, der pH-Wert erneut eingestellt und verdunstetes Wasser durch entmineralisiertes Wasser ersetzt. Zur Bestimmung der am Bioschlamm absorbierten Substanz werden zwei Proben auf ihren CSB-Gehalt analysiert, eine unfiltrierte Probe wird direkt aus dem gut durchmischten Reaktiongefäß entnommen und gemessen. Für die zu filtrierende Probe wird ein aliquoter Teil, zum Beispiel 40 ml, entnommen, absetzen lassen und über ein Millipor 2,5 μ Filter (z. B. Millipor Millex GS) filtriert. Die klare Lösung wird ebenfalls auf ihren CSB-Gehalt analysiert.

Berechnung

Um CSB-Gehalt der Substanz zu ermitteln, der an der Biomassen adsorbiert ist, wird vom CSB in der Probelösung mit Biomasse der CSB-Wert der filtrierten Probe und der Blindwert mit Biomasse abgezogen.

P1 unfiltrierte Probelösung mit Schlamm
P2 filtrierte Probelösung
Bld unfiltrierter Blindwert
ad adsorbierte Substanz

$$P1 - P2 - Bls = ad$$

Wird von der filtrierten Probe P2 der filtrierte Blindwert abgezogen, so erhält man die gelöste Substanz.

B1 filtrierter Blindwert
S gelöste Substanz

$$P2 - Bl = S$$

Adsorbierte und gelöste Substanz zusammen ergeben den Anfangsgehalt an abzubauendem Stoff bzw. den Gehalt zur Zeit. Der biologische Abbau η berechnet sich

A Anfangswert
η biologischer Abbau

$$\eta = \frac{A-(ad+s)}{A}\ 100$$

**Beispiele**

**Beispiel 1 - Synthese von Stärkemaleat**

50 g (= 0,276 mol) Aeromyl 115 (physikalisch modifizierte, kaltwasserlösliche Stärke der Firma Südstärke; Restfeuchte = ca. 12 %) werden in 400 ml Wasser gelöst. Der pH wird mit 3 N NaOH auf 8 eingestellt und während der Reaktion konstant gehalten. Bei einer Reaktionstemperatur von 0 °C werden 27,1 g (= 0,276 mol) festes Maleinsäureanhydrid über einen Zeitraum von 2 h zugegeben. Es wird weitere 3 h bei 25 °C gerührt (Nachreaktion). Der Substitutionsgrad (DS(NaOH)) beträgt 0,81.
Die Reaktionslösung wird einrotiert und im Vakuumtrockenschrank nachgetrocknet.
SRV = 19,1 g/g; A(NaCl) = 13,2 ml/g

**Beispiel 2 - Synthese von Stärkemaleat**

Wie Beispiel 1, die Reaktionslösung wird jedoch gefriergetrocknet.
SRV = 16,3; A(NaCl) = 14,8 ml/g; lösliche Anteile = 35,2 %

**Beispiel 3**

Das Produkt aus Beispiel 2 wird mit 5 % einer Fällungskieselsäure (FK 500 LS, Degussa AG) vermischt.
A(NaCl) = 21,2 ml/g, AUL(NaCl) = 5,4 ml/g

**Beispiel 4 - Synthese von Stärkemaleat**

50 g (= 0,269 mol) Aeromyl 115 (physikalisch modifizierte, kaltwasserlösliche Stärke der Firma Südstärke; Restfeuchte = ca. 12 %) werden in 400 ml Wasser gelöst. Der pH wird mit 3 N NaOH auf 8 eingestellt und während der Reaktion konstant gehalten. Bei einer Reaktionstemperatur von 0 °C werden 13,2 g (= 0,135 mol) festes Maleinsäureanhydrid über einen Zeitraum von 2 h zugegeben. Es wird weitere 3 h bei 25 °C gerührt (Nachreaktion). Die Substitutionsausbeute beträgt 83,1 % und der Substitutionsgrad dementsprechend 0,42.
Die Reaktionslösung wird durch Eingießen der Reaktionslösung in Aceton ausgefällt und im Vakuumtrockenschrank bei Temperaturen < 50°C getrocknet.
SRV = 11,0 g/g; A(NaCl) = 14,9 ml/g, AUL(NaCl) = 7,6 ml/g

**Beispiel 5 - Synthese eines gemischten Stärkeesters**

50 g (= 0,276 mol) Aeromyl 115 (physikalisch modifizierte, kaltwasserlösliche Stärke der Firma Südstärke; Restfeuchte = ca. 12 %) werden in 400 ml Wasser gelöst. Der pH wird mit 3 N NaOH auf 8 eingestellt und während der Reaktion konstant gehalten. Bei einer Reaktionstemperatur von 0 °C wird eine Mischung aus 13,36 g (0,136 mol) festem Maleinsäureanhydrid und aus 13,61 g (0,136 mol) festem Bernsteinsäureanhydrid über einen Zeitraum von 2 h zugegeben. Es wird weitere 3 h bei 25 °C gerührt (Nachreaktion). Der Substitutionsgrad (DS(NaOH)) beträgt 0,80.)
Die Reaktionslösung wird einrotiert und im Vakuumtrockenschrank nachgetrocknet.
SRV = 15,5 g/g; A(NaCl) = 4,6 ml/g

**Beispiel 6**

Das Produkt aus Beispiel 2 wird mit 5 % einer Fällungskieselsäure (FK500LS, Degussa AG) vermischt.
A(NaCl) = 24,8 ml/g; AUL(NaCl) = 3,8 ml/g

**Vergleichsbeispiel 1 - Synthese von Stärkesuccinat**

50 g (= 0,278 mol) Aeromyl 115 (physikalisch modifizierte, kaltwasserlösliche Stärke der Firma Südstärke; Restfeuchte = 11,9 %) werden in 400 ml Wasser gelöst. Der pH wird mit 3 N NaOH auf 9 eingestellt und während der Reaktion konstant gehalten. Bei einer Reaktionstemperatur von 25 °C werden 31,5 g (= 0,315 mol) festes Bernsteinsäureanhydrid über einen Zeitraum von 2 h zugegeben. Es wird weitere 3 h bei 25 °C gerührt und anschließend wird das Produkt durch Zugabe von Aceton ausgefällt. Das Produkt wird abfiltriert und bei RT im Vakuumtrockenschrank getrocknet. Die Substitutionsausbeute beträgt 81 % und der Substitutionsgrad 0,92.
SRV - nicht bestimmbar, da das Produkt aufgrund seiner Wasserlöslichkeit aus dem Nylonsiebbeutel fließt.
Weitere Ausführungsformen und Vorteile der Erfindung ergeben sich aus den nachfolgenden Patentansprüchen.

## Patentansprüche

1. Verfahren zur Herstellung eines quellbaren Stärkeesters, der zu mehr als 50 Gew.-% aus in Wasser unlöslichen Anteilen besteht und ein Rückhaltevermögen für 0,9 gew.-%ige wässrige NaCl-Lösung von > 500 % aufweist, jeweils bezogen auf das Gewicht des trockenen Stärkeesters, wobei das Rückhaltevermögen dadurch bestimmt wird, daß man 0,1 g des in einen Nylonbeutel mit 52 μm Maschenweite eingeschweißten Stärkeesters für 30 min in einer 0,9 %igen NaCl-Lösung quellen läßt, den Beutel anschließend 5 min bei 1400 rpm zentrifugiert und danach eine ggf. resultierende Gewichtszunahme gravimetrisch bestimmt, bei welchem Verfahren Stärke oder modifizierte Stärke in einer einstufigen wässrigen Reaktion mit einem Carbonsäureanhydrid oder einer Mischung von Carbonsäureanhydriden bei einem pH-Wert von 7 bis 11 und einer Temperatur von 0 bis 40 °C umgesetzt wird, wobei der pH-Wert durch Zugabe von wässriger Alkali-Lösung mit einer Konzentration von ca. 10 bis 50 Gew.-% im angegebenen Bereich gehalten wird, und wobei als Carbonsäureanhydrid ein cyclisches ungesättigtes Anhydrid oder eine Mischung von Carbonsäureanhydriden eingesetzt wird, die ein solches cyclisches ungesättigtes Anhydrid aufweist.

2. Verfahren nach Anspruch 1,
**dadurch gekennzeichnet,**
daß als Base Natriumhydroxid, Kaliumhydroxid, Calciumhydroxid, Ammoniumhydroxid, Magnesiumhydroxid und/oder Natriumcarbonat eingesetzt wird.

3. Verfahren nach einem der Ansprüche 1 oder 2,
**dadurch gekennzeichnet,**
daß als Carbonsäureanhydrid Maleinsäureanhydrid verwendet wird oder eine Mischung, die Maleinsäureanhydrid aufweist.

**Claims**

1. A process for preparing a swellable starch ester, more than 50 wt.% of which consists of fractions which are insoluble in water and which has a retention capacity for 0.9 wt.% aqueous NaCl solution of > 500 %, each being with respect to the weight of dry starch ester, wherein the retention capacity is determined by permitting 0.1 g of the starch ester, sealed in a nylon bag with a 52 µm mesh, to swell up in a 0.9 % strength NaCl solution for 30 min, then centrifuging the bag for 5 min at 1400 rpm and then determining any optionally resulting increase in weight gravimetrically, in which process starch or modified starch is reacted in a one-stage aqueous reaction with a carboxylic anhydride or a mixture of carboxylic anhydrides at a pH of 7 to 11 and a temperature of 0 to 40°C, wherein the pH is held within the said range by adding aqueous alkali solution with a concentration of about 10 to 50 wt.%, and wherein a cyclic unsaturated anhydride or a mixture of carboxylic anhydrides which contains such a cyclic unsaturated anhydride is used as a carboxylic anhydride.

2. A process according to Claim 1, characterised in that sodium hydroxide, potassium hydroxide, calcium hydroxide, ammonium hydroxide, magnesium hydroxide and/or sodium carbonate is used as a base.

3. A process according to one of Claims 1 or 2,
characterised in that maleic anhydride or a mixture which contains maleic anhydride is used as a carboxylic anhydride.

**Revendications**

1. Procédé de préparation d'un ester d'amidon gonflable qui est constitué à plus de 50% en poids de fractions insolubles dans l'eau et possède un pouvoir de rétention de plus de 500% pour une solution aqueuse de NaCl à 0,9% en poids, respectivement par rapport au poids de l'ester d'amidon sec, le pouvoir de rétention étant déterminé en faisant gonfler 0,1 g de l'ester d'amidon enfermé dans un sachet de Nylon scellé d'une largeur de mailles de 52 µm pendant 30 minutes, en centrifugeant ensuite le sachet pendant 5 minutes à 1400 tours et en déterminant ensuite l'augmentation de poids éventuellement obtenue par voie gravimétrique, procédé dans lequel on fait réagir de l'amidon ou de l'amidon modifié, dans une réaction aqueuse en une seule étape, avec un anhydride d'acide carboxylique ou un mélange d'anhydrides d'acides carboxyliques, à une valeur du pH de 7 à 11 et à une température de 0 à 40°C, réaction dans laquelle la valeur du pH est maintenue dans la plage indiquée par addition d'une solution aqueuse d'alcali d'une concentration d'environ 10 à 50% en poids et on utilise comme anhydride d'acide carboxylique un anhydride cyclique insaturé ou un mélange d'anhydrides d'acides carboxyliques qui comprend un tel anhydride cyclique insaturé.

2. Procédé selon la revendication 1, caractérisé en ce qu'on utilise comme base de l'hydroxyde de sodium, de l'hydroxyde de potassium, de l'hydroxyde de calcium, de l'hydroxyde d'ammonium, de l'hydroxyde de magnésium et/ou du carbonate de sodium.

3. Procédé selon l'une quelconque des revendications 1 ou 2, caractérisé en ce qu'on utilise comme anhydride d'acide carboxylique un anhydride d'acide maléique ou un mélange qui comprend de l'anhydride d'acide maléique.